Europäisches Patentamt

(19) European Patent Office

Office européen des brevets

(11) Publication number: **0 048 181**
**A2**

(12) # EUROPEAN PATENT APPLICATION

(21) Application number: **81304281.9**

(22) Date of filing: **17.09.81**

(51) Int. Cl.³: **A 61 B 3/12**

(30) Priority: **17.09.80 US 187900**

(43) Date of publication of application:
**24.03.82 Bulletin 82/12**

(84) Designated Contracting States:
**DE FR GB**

(71) Applicant: **Fukai, Michael Edwin**
**1017 South Boulder Road**
**Louisville Colorado 80027(US)**

(72) Inventor: **Fukai, Michael Edwin**
**1017 South Boulder Road**
**Louisville Colorado 80027(US)**

(74) Representative: **Bowman, Paul Alan et al,**
**LLOYD WISE, TREGEAR & CO. Norman House 105-109**
**Strand**
**London WC2R OAE(GB)**

(54) Binocular indirect ophthalmoscope with camera.

(57) An improved portable ophthalmoscope of the binocular
indirect type wherein the ophthalmoscope has a camera
mounted thereon for photographing the fundus of the eye of
a patient when the ophthalmoscope is worn on the head of a
clinician. The ophthalmoscope has an adjustable head
assembly providing a support for an observation housing
having reflecting surfaces permitting a stereo scopic view of
the fundus of the patient's eye. The camera is mounted at the
front of the head assembly near the observation housing and
the camera can be actuated with a foot switch or other
actuator. A light source is also carried by the head assembly
and has means for directing light into the patient's eye. Light
reflected from the fundus is received in the observation
housing. In several embodiments of the invention, the
camera is mounted above the observation housing and
receives a part of the reflected light from the fundus while
the remainder of the reflected light can be simultaneously
received for binocular viewing by the clinician. In another
embodiment, the camera is at one side of the observation
housing and receives light rays reflected laterally to the one
side. Side mirrors in the observation housing permit binocu-
lar viewing forwardly of the ophthalmoscope, one of the side
mirrors being half-silvered or pivotally mounted in the
housing to permit reflected light to enter the camera at the
same time as or alternately with binocular viewing of the eye
of the patient.

FIG. 1

8140-1A/FFFF03C

## BINOCULAR INDIRECT OPHTHALMOSCOPE WITH CAMERA

This invention relates to improvements in ophthalmoscopes and, more particularly, to a binocular indirect ophthalmoscope which has the capability of photographing the fundus of the eye.

## BACKGROUND OF THE INVENTION

Binocular indirect ophthalmoscopes using cameras to photograph the fundus of the eye have been known in the past. However, the various attempts to use a camera on an ophthalmoscope of this type have generally not been satisfactory for one reason or another. Generally, such attempts have resulted in apparatus which is complex in construction and expensive to produce and to maintain. Moreover, early ophthalmoscopes of this type have been heavy and cumbersome, producing strain on the users, causing a loss of portability and causing many clinicians to resort to the use of sophisticated bench type retinal cameras and to the use of the well-known technique of drawing images of the fundus by hand while viewing into the eye, a procedure which is laborious and time-consuming and thereby costly to the patient. For these reasons, a need has arisen for an improved binocular indirect ophthalmoscope having the capabilities of photographing the fundus of the eye while the ophthalmoscope is comfortably worn on the head of a clinician.

Prior disclosures in the field of retinal photography include the following U.S. Patents:

| | |
|---|---|
| 2,327,612 | 3,698,099 |
| 3,089,398 | 3,851,954 |
| 3,217,622 | 3,914,032 |
| 3,609,016 | 4,018,514 |
| 3,614,214 | 4,149,787. |

## SUMMARY OF THE INVENTION

The present invention satisfies a need for an improved ophthalmoscope of the binocular indirect type which is portable and can be comfortably worn on the head of a clinician for not only viewing the interior of an eye of a patient but also for photographing the fundus of the eye. To
this end, the present invention includes a head assembly
which is adjustable in size, the head assembly defining a
support for an observation housing containing reflecting
surfaces and a front opening for permitting binocular viewing forwardly of the housing and into the eye of the
patient. A camera is carried by the head assembly and is
optically located with reference to the reflecting surfaces
in the observation housing so that light reflected from the
fundus of the eye can enter the housing and be directed into
the camera to expose the film therein.

In several embodiments of the invention, the
camera is above the observation housing and the camera
shutter can be actuated while the eye of the patient is
simultaneously being viewed in binocular fashion by the
clinician. In one of these embodiments, a light source is
mounted near the rear of the head assembly and in another of
these embodiments, the light source is mounted at the front
of the head assembly, just below the camera.

In a further embodiment, the camera is mounted at
one side of the observation housing and a light source is
above the observation housing. Thus, light entering the
housing can be directed laterally to the camera for exposing
the film therein when the camera shutter is actuated. In
the latter embodiment, the housing has a reflecting surface
which can either be half-silvered or pivotally mounted in
the housing to allow light entering the housing from the eye
of the patient to be directed into the camera. An actuator
associated with the pivotal reflecting surface is selectively energized by a foot-operated switch or other suitable
actuation means to pivot the reflecting surface out of the
path of the light traveling to the camera.

The ophthalmoscope of the present invention is
lightweight in construction to minimize strain on the
wearer. Also, the camera, the observation housing and the
light source are suitably located to distribute the weight
about the head of the wearer to further minimize strain. By
placing the camera adjacent to the observation housing, the length of the path over which light rays pass is minimized to reduce the number of required optical elements and thereby the weight of the system to thereby decrease production and maintenance costs.

The primary object of the present invention is to provide an improved portable ophthalmoscope of the binocular indirect type wherein the ophthalmoscope has a camera associated therewith for photographing the fundus of the eye of a patient when the ophthalmoscope is comfortably worn on the head of a clinician.

Other objects of this invention will become apparent as the following specification progresses, reference being had to the accompanying drawings for an illustration of several embodiments of the invention.

## IN THE DRAWINGS

Fig. 1 is a side elevational view of a first embodiment of an ophthalmoscope of the present invention;

Fig. 2 is a front perspective view of the opthalmoscope of Fig. 1;

Fig. 3 is a schematic view of the ophthalmoscope of Figs. 1 and 2 showing its binocular viewing capability;

Fig. 4 is an internal view of the observation housing of the opthalmoscope of Figs. 1 and 2;

Fig. 5 is a perspective view of the bracket for holding a beam splitter in the observation housing of Fig. 4;

Fig. 6 is a side elevational view of the adapter tube carried by the observation housing for directing light therefrom to a camera thereabove;

Fig. 7 is a front elevational view of a camera used with the ophthalmoscope of Figs. 1 and 2;

Fig. 8 is a view similar to Fig. 1 but showing a modification of the ophthalmoscope of Figs. 1 and 2;

Fig. 9 is a view similar to Fig. 2 but showing a front perspective view of the modified ophthalmoscope of Fig. 8;

Fig. 10 is an enlarged, schematic view of the condensing lens assembly for use with a fiber optic bundle for directing light from a light source to the eye of a patient;

Fig. 11 is a view similar to Fig. 2 but showing a second embodiment of the ophthalmoscope of the present invention;

Fig. 12 is a bottom schematic view of the ophthalmoscope of Fig. 11;

Fig. 13 is a view similar to Figs. 1 and 8 but showing a preferred embodiment of the ophthalmoscope of the present invention;

Fig. 14 is a front elevational view of the ophthalmoscope of Fig. 13;

Fig. 15 is an enlarged, schematic view of the light source of the ophthalmoscope of Figs. 13 and 14;

Fig. 16 is a schematic view of the light path from the eye of a patient to the camera of the ophthalmoscope of Figs. 13 and 14, showing the improved Keplerian lens system of the camera;

Fig. 16a is a view similar to Fig. 16 but showing the use of lenses forming a Galilean telescopic system with the lenses of the camera; and

Fig. 17 and 18 are front and side views, respectively, of a prism for use with the ophthalmoscopes of Figs. 1 and 2 and Figs. 13 and 14.

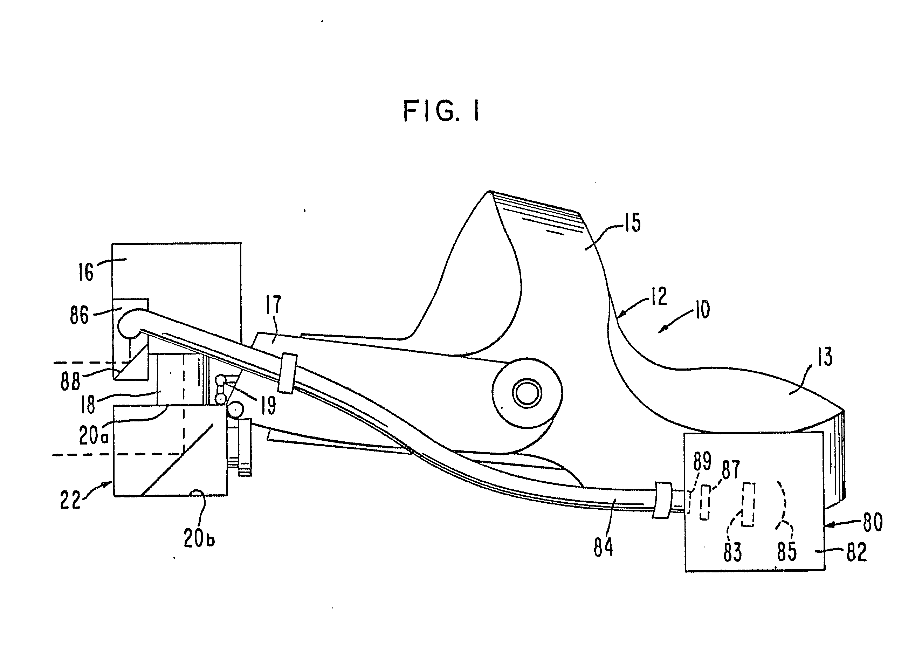

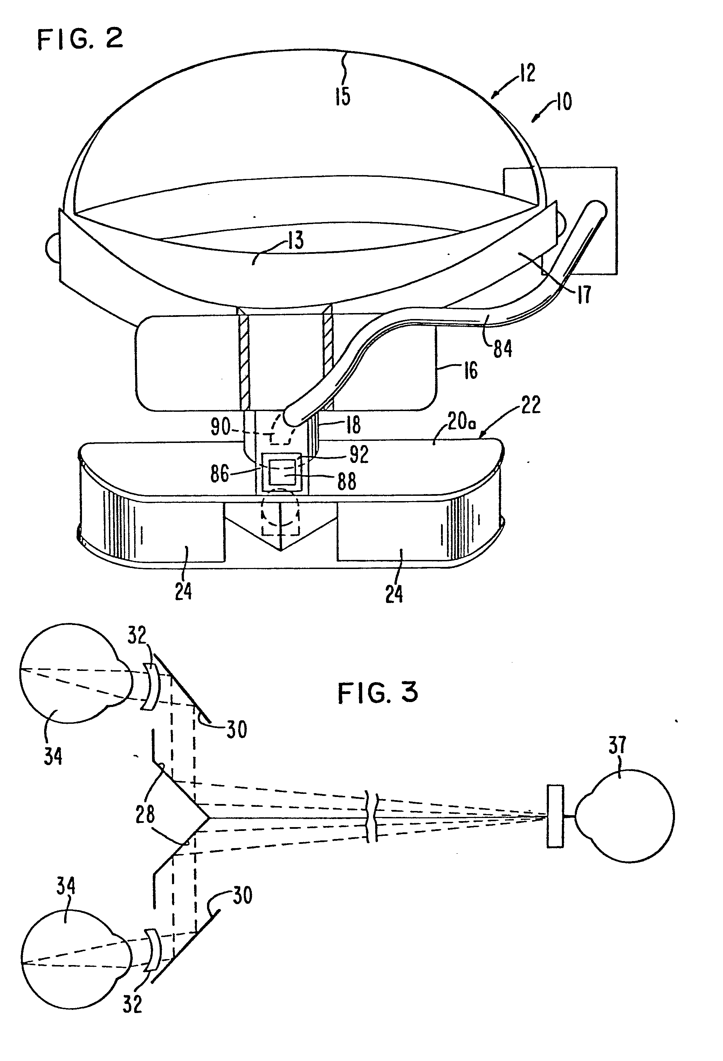

The first embodiment of the binocular indirect ophthalmoscope of the present invention is broadly denoted by the numeral 10 and is shown in assembled form in Figs. 1 and 2. Ophthalmoscope 10 includes an adjustable head assembly 12 which is adapted to be worn on the head of a clinician using the ophthalmoscope. Assembly 12 has a head band 13 for encircling the head, a strap 15 integral with head band 13, and a semi-circular band 17 carried at rear ends thereof on the sides of band 13 and extending toward the front end thereof as shown in Fig. 2. The forward part of band 17 is secured by an attachment device 19 to an observation housing 22 below a camera 16. Band 17 may be mounted on band 13 so that it can pivot up and down with reference to band 13, if desired, to adjust the vertical position of housing 22 and camera 16. However, band 17 is generally rigidly secured to band 13 so that camera 16 is at all times in a fixed position with respect thereto. Camera 16 and housing 22 are shown in Fig. 2 as being forwardly of their normally operative positions relative to band 17.

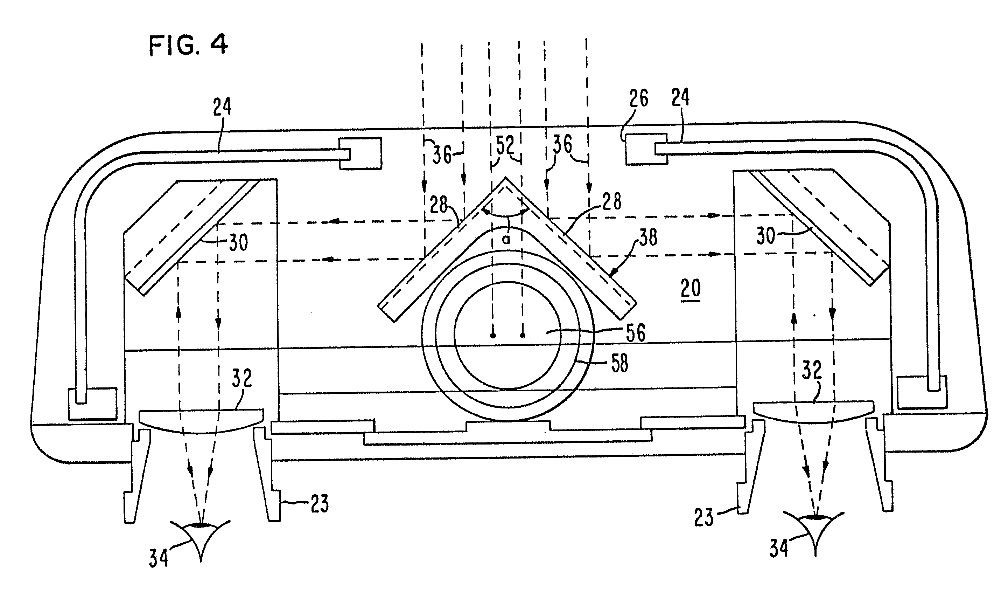

The housing of camera 16 has a tube 18 secured thereto and extending downwardly therefrom. The tube has an upper open end surrounding the lens mount of the camera. The tube extends through the top wall 20a of an observation housing 22 and the lower end of the tube 18 is secured in any suitable manner to the bottom 20b of the observation housing 22. Housing 22 is provided with means therein for permitting binocular vision forwardly of ophthalmoscope 10 when the latter is worn on the head of the user and when the user's eyes are optically aligned with ocular members 23 (Fig. 4) at the rear of housing 22.

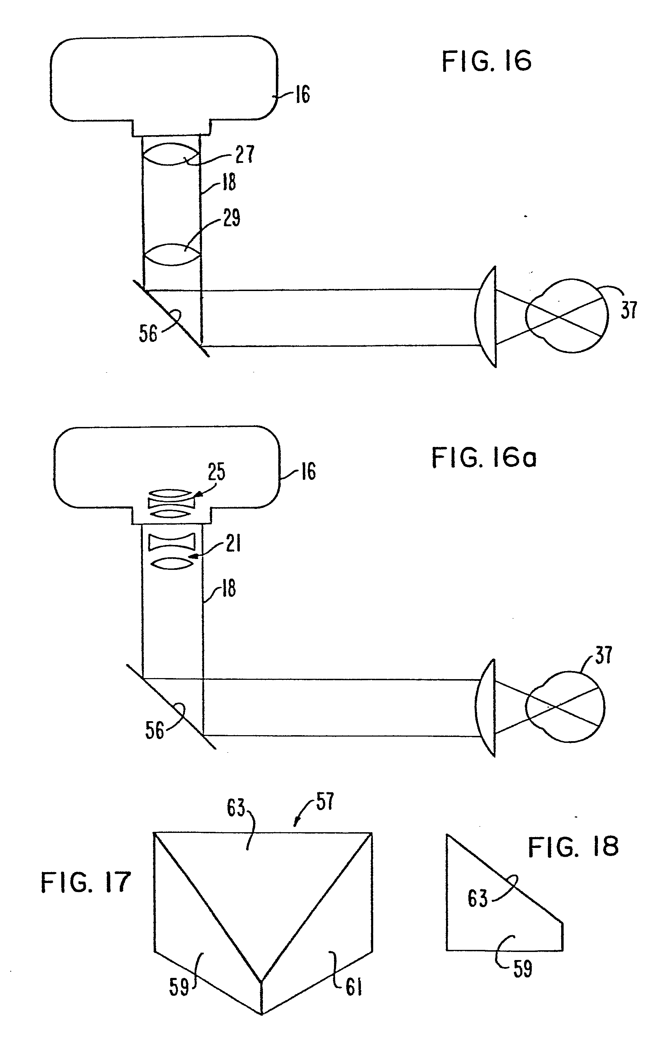

Tube 18 contains a lens assembly 21 defining a Galilean telescopic system in series with the camera lens assembly 25 (Fig. 16a). This arrangement of lenses magnifies the image of the patient's fundus and focusses this image on the film plane of the camera.

It is possible to replace the lens arrangement of Fig. 16a with the lens arrangement of Fig. 16 in which a pair of spaced positive lenses 27 and 29 are mounted in tube 18 and form a Keplerian lens system which functions in the same manner as the lens system in Fig. 16 but with few parts. This feature reduces the weight of the ophthalmoscope and provides a greater field of view, minimized aberrations at higher magnifications and improved imagery.

Housing 22 is seen in more detail in Fig. 4 and includes a pair of spaced front wall sections 24 defining a front opening 26 through which light rays pass, the light rays emanating from the fundus of the eye 37 (Fig. 3) of a patient to be photographed. The incoming light rays entering housing 22 through opening 26 strike a pair of angled first reflecting surfaces defining a beam splitter, such as the surfaces of a pair of mirrors 28, and are directed laterally onto angled second reflecting surfaces, such as mirrors 30, near the opposed sides of housing 22 and interiorly thereof. Mirrors 30 reflect the light rays through lenses 32 of ocular members 23 and into the eyes 34 of the user of ophthalmoscope 10 when assembly 12 is mounted on the head of the user. Ocular members 32 may be slidably mounted on housing 22 for lateral movement relative thereto to permit adjustment of the spacing between members 32.

Fig. 4 shows light rays 36 from the eye 37 of the patient to be photographed, the rays striking mirrors 28, then mirrors 30, passing through lenses 32 and into eyes 34. The binocular viewing capability of housing 22 with its reflecting surfaces is shown schematically in Fig. 3.

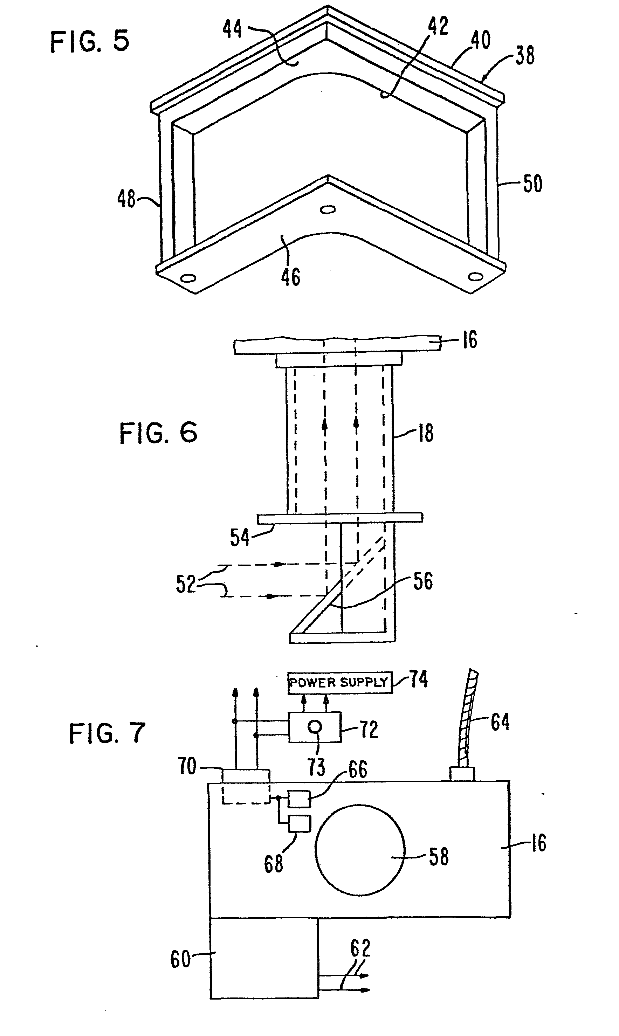

Mirrors 28 are mounted on an L-shaped bracket 38 which is secured to bottom 20 and shown in detail in Fig. 5. Bracket 38 includes a frame-like body 40 having a central opening 42 which extends between upper and lower L-shaped members 44 and 46 and between a pair of side members 48 and 50. Opening 42 is completely unobstructed in the space between side members 48 and 50. Mirrors 28 are placed in opening 42 and make an angle a (Fig. 4) of approximately 90°.

At least the front portions of mirrors 28 are half-silvered so that the mirrors will pass light rays 52 rearwardly of bracket 38 in the manner shown in Fig. 4. These light rays 52, after passing through mirrors 28, enter a front opening 54 (Fig. 6) in the lower end of tube 18 and strike an oval mirror 56 mounted at an angle as shown in Fig. 6 to cause reflection of rays 52 upwardly and into and through the lens arrangement of Fig. 16 or Fig. 16a and into camera 16. The light rays then pass through the aperture and the plane of the shutter of the camera and onto the film in the camera when the shutter is tripped for exposing the film with an image of the fundus of the eye of a patient being examined.

While mirrors 28 and 56 have been described with respect to the reflection of incoming light rays 36 and 52, these mirrors could be replaced by a light reflecting device 57 (Figs. 17 and 18) having three reflecting surfaces 59, 61 and 63. This device can be a single 45° prism having angled front reflecting surfaces 59 and 61 for reflecting rays 36 and inclined upper reflecting surface 63 extending upwardly and rearwardly from the front of the prism for reflecting light rays 52 upwardly. Location of the prism with reference to tube 18 may be slightly different from that shown in Fig. 4 to accommodate the prism. Instead of a prism, an aluminum block may be used in which the block can have flat surfaces for mounting three plane mirrors which define surfaces 59, 61 and 63.

Camera 16 is shown in more detail in Fig. 7. Its own lens is used if the lens arrangement of Fig. 16a is used. If the lens arrangement of Fig. 16 is used the camera lens is not used.

The camera has a motor drive mechanism 60 for automatically advancing the film in the camera after each exposure of the film. Electrical leads 62 from mechanism 60 are directed to a power supply whose output typically is 3.5 volts d.c.

Camera 16 also has a shutter cable 64 which trips the shutter of the camera when a certain action occurs. The shutter cable can be operated by a foot switch or other suitable actuator to permit the user of opthalmoscope 10 to have both hands free when a photograph is to be taken.

Camera 16 has an electrically actuated shutter speed setting mechanism 66, an electrically actuated aperture adjusting mechanism, and a signal input means to mechanisms 66 and 68. The camera is provided with electronics 70 coupled electrically to mechanisms 66 and 68, and the input to electronics 70 includes a manually operated control 72 fed by a power supply 74 so that a d.c. voltage signal can be applied from the power supply 74 through control 72 to set the shutter speed and aperture adjusting mechanisms 66 and 68 before taking a particular photograph of the fundus of the eye. Typically, the maximum output of power supply 74 is 5 volts d.c. and control 72 has a rotatable knob 73 which permits variations in the range of 0-5 volts applied to mechanisms 66 and 68. The higher the voltage, the faster the shutter speed and the greater diameter of the aperture of the camera.

Opthalmoscope 10 has a light source 80 carried in a housing 82 secured to head assembly 12 in any suitable manner, preferably near the rear end of band 13. Generally, the light source will include a light bulb 83 (Fig. 1) in housing 82 which is electrically actuated from a power source (not shown) having an output voltage typically in the range of 6-12 volts AC. The light source preferably has a reflecting mirror 85 at the rear thereof and an infra-red filter 87 forwardly thereof. Light from bulb 83 will pass into one end 89 of a fiber optics bundle 84 extending forwardly along band 13 to a tubular extension 86 in front of camera 16, extension 86 having focusing lenses (not shown in Fig. 2) for focusing the light on an inclined mirror 88 therein. The light from the forward end 90 (Fig. 2) of fiber optics bundle 84 leaves the bundle, passes through the focusing lenses and strikes mirror 88 and is reflected forwardly from extension 86 through a front opening 92 therein. The light will then proceed forwardly and the angle of the mirror 88 can be adjusted so that the light will enter the eye of the patient and strike the fundus of the eye. The reflected light from the fundus will be directed back to the opthalmoscope in the manner disclosed above with respect to Figs. 3 and 4.

Light source 80 is mounted near the rear of opthalmoscope 10 to balance the weight of camera 16 and housing 22 at the front of the opthalmoscope. This will reduce the strain on the wearer. Also, by placing the camera at the front of the opthalmoscope, light rays to be photographed can take the shortest possible path from the patient to the camera to thereby eliminate the need for additional mirrors, prisms, lenses and other optical components. This results in a generally lightweight construction for the opthalmoscope and further adds to the reduction of strain placed on the wearer.

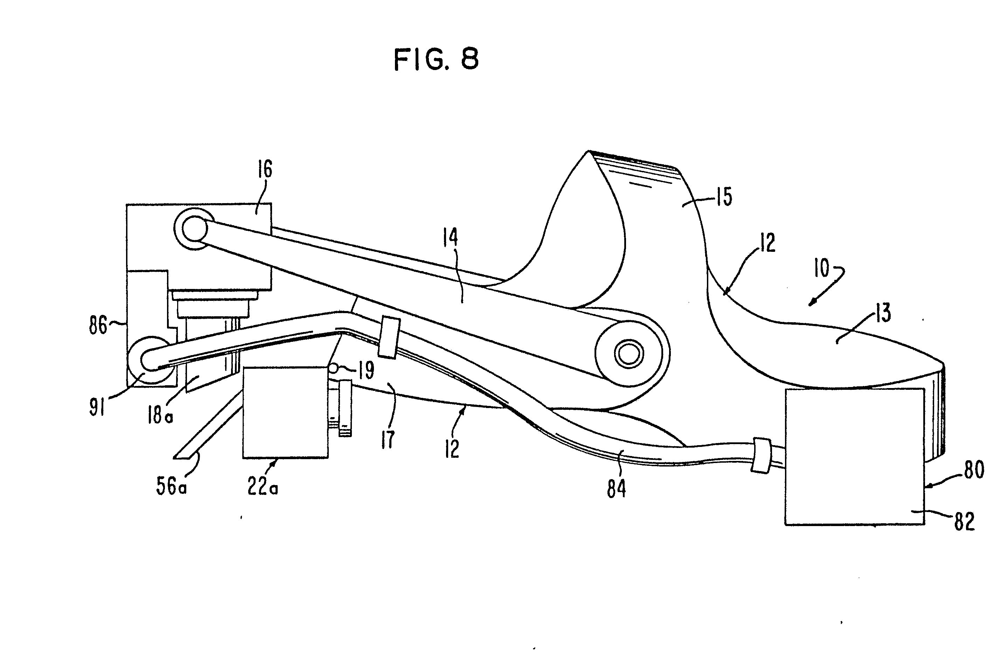

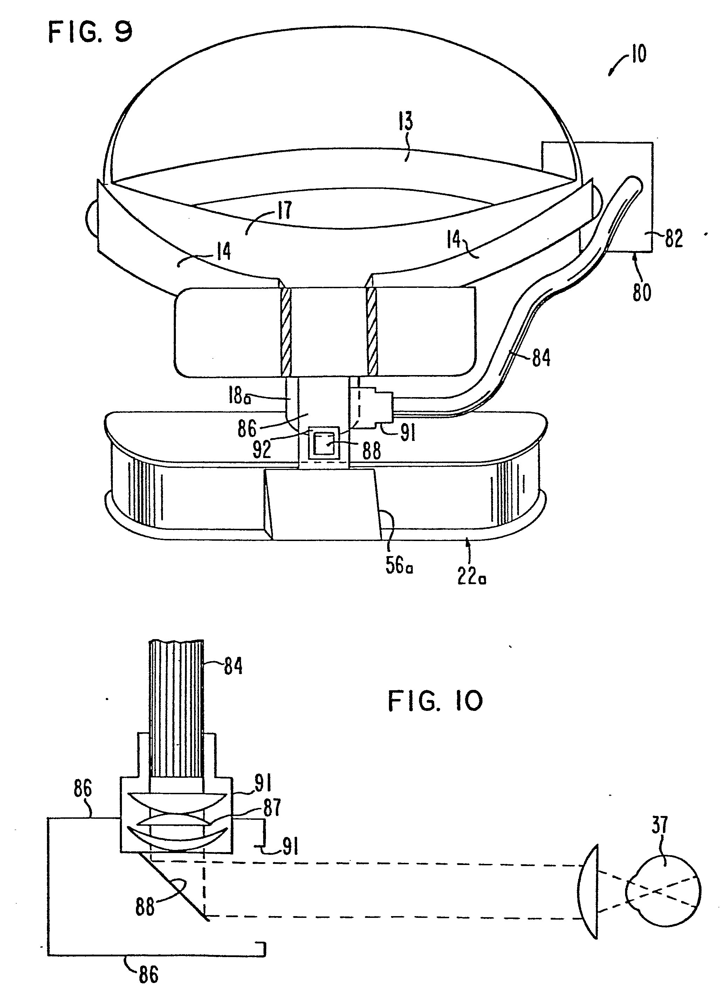

Figs. 8 and 9 show opthalmoscope 10 in a slightly modified form. The modification involves an observation housing 22a which is coupled by attachment means 19 to front band 17. Camera 16 is coupled to the front ends of a pair of arms 14 which are secured at their rear ends to the sides of band 13. The housing 22a has a half-silvered mirror 56a in front and externally of the housing for directing light upwardly into the open lower end of a vertical tube 18a and then into camera 16. Mirror 56a also passes light into housing 22a where the light strikes mirrors 28 and then is reflected toward and onto mirrors 30 in the manner shown and described above with respect to Figs. 3 and 4.

Fig. 10 shows the way in which the fiber optics bundle 84 is coupled to extension 86 for the modified opthalmoscope of Figs. 8 and 9 and for the opthalmoscope of Figs. 1 and 2, if desired. A condensing lens assembly 87 is mounted in a lateral tube 91 between the end of bundle 84 and mirror 88 for collimating the light rays from the bundle before the light rays exit through opening 90. Tube 91 is coupled to extension 86 in any suitable manner.

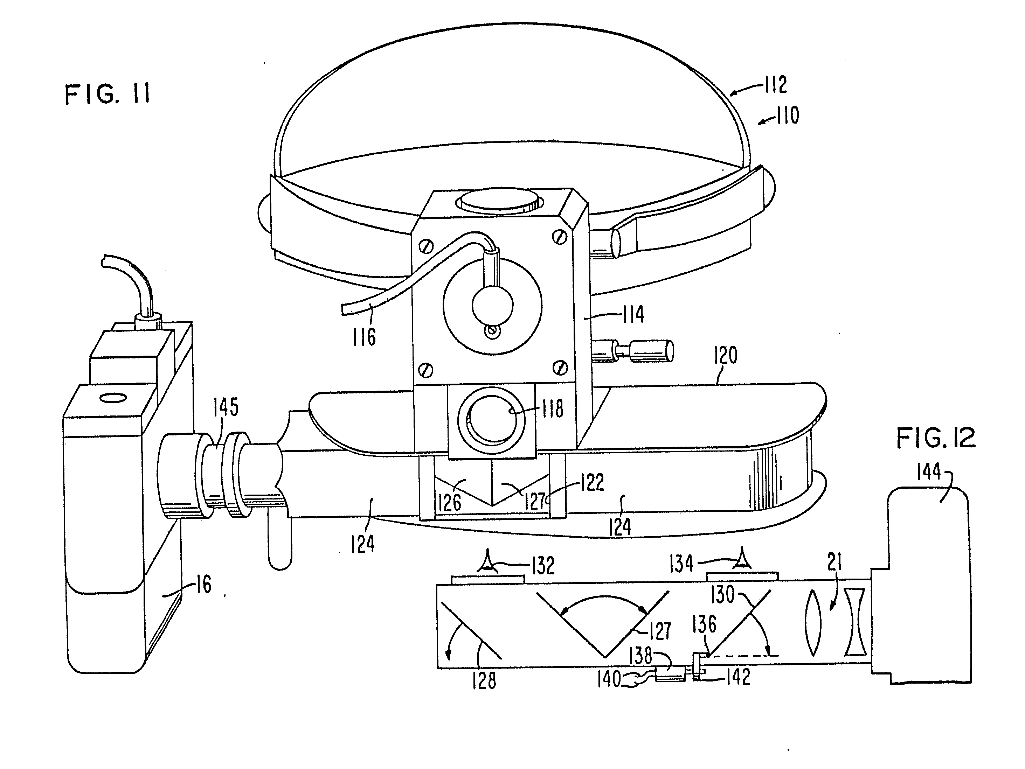

Another embodiment of the opthalmoscope of the present invention is shown in Figs. 11 and 12 and is denoted by the numeral 110. Opthalmoscope 110 includes an adjustable head assembly 112 to be worn on the head of the user of the opthalmoscope. The front end of head assembly 112 supports a housing 114 containing a light source electrically actuated by a power supply (not shown) through electrical leads of a cable 116 coupled to the light source itself. The light from the light source is directed out of housing 114 through an exit port 118 and is directed forwardly into the eye of the patient whose fundus is to be photographed. Housing 114 has mirrors or other optical elements (not shown) to direct the light from the light source itself to and through port 118.

An observation housing 120 is coupled to and supported by the lower end of housing 114. Housing 120 has a front opening 122 for receiving reflected light from the fundus of the eye illuminated by the light leaving port 118.

Housing 120 has opaque front walls 124 on opposite sides of opening 122 and a pair of reflecting surfaces 126 and 127 rearwardly of opening 122, surfaces 126 being angled with reference to each other to split the incoming light rays into two parts. Surfaces 126 and 127 can be the surfaces of mirrors or a prism, whichever is desired. Light reflected by surfaces 126 are directed laterally and strike reflecting surfaces or mirrors 128 and 130, and mirrors 128 and 130 then reflect the light into the eyes 132 and 134 (Fig. 12) of the wearer of opthalmoscope 110.

Mirror 130 is either half-silvered or is shiftably mounted in housing 120. If shiftably mounted mirror 130 is pivotally mounted by a pin 136 in housing 120 for rotation about a generally vertical axis from the full line position thereof shown in Fig. 12 to the dashed line position when an actuator 138 coupled to mirror 130 is energized. This actuator can be of mechanical construction for manual actuation by a longitudinally shiftable cable or it can be a solenoid which is electrically actuated when a voltage is impressed upon leads 140 coupled to the coil of the solenoid. The armature or other shiftable portion of the actuator is pivotally mounted to a crank 142 coupled with pin 136 to cause rotation of mirror 130 in a desired direction when the actuator is energized. Typically, the actuator is energized only in one direction, such as into the dashed line position of Fig. 12, there being a spring (not shown) for returning mirror 130 to the full line position of Fig. 12 when the actuator is deenergized.

When mirror 130 is in the dashed line position of Fig. 12, light reflected from the corresponding reflecting surface 127 will pass directly into a camera 144 secured by a tube 145 to one end of housing 120. The camera is substantially the same in construction and operates in the same way as described above with respect to opthalmoscope 10.

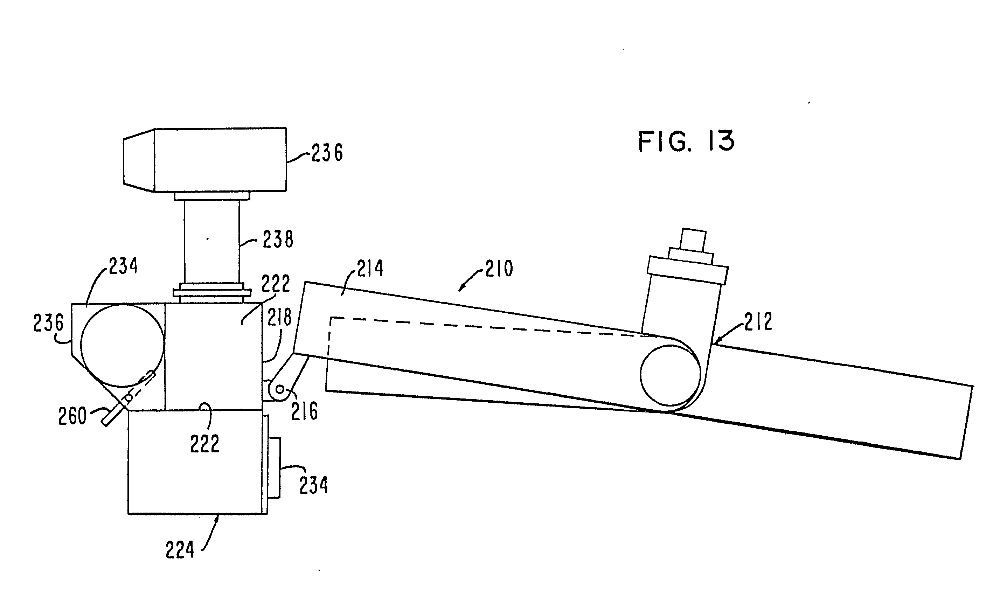

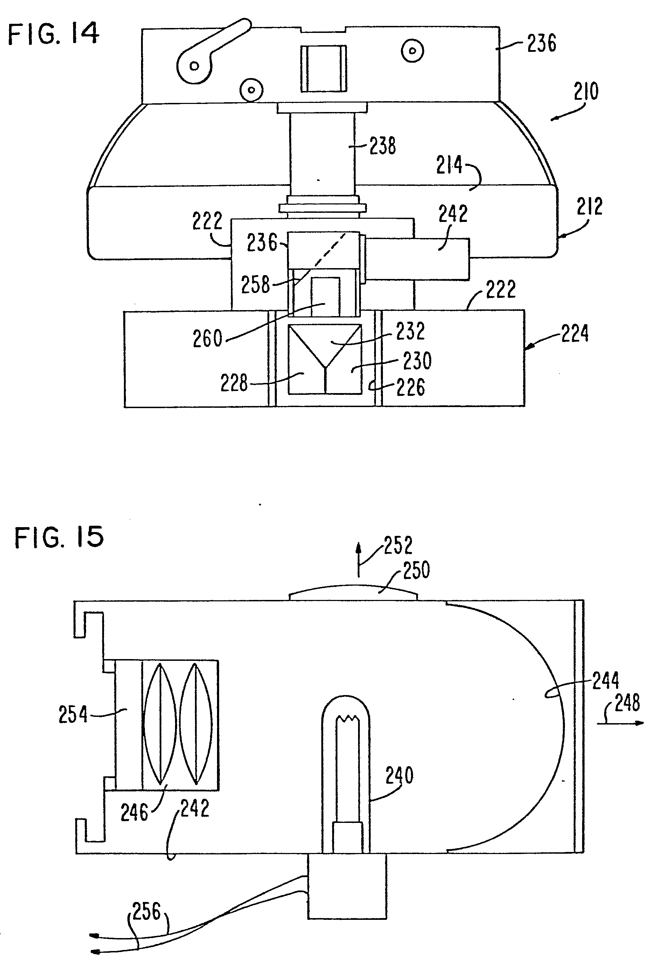

Another embodiment of the opthalmoscope of the present invention is shown in Figs. 13 and 14 and is broadly denoted by the numeral 210. Opthalmoscope 210 includes an adjustable head assembly 212 having a generally semi-circular band 214 at the front portion thereof. An attachment means 216 couples band 214 to the rear face 218 of a hollow housing 220 mounted on the top wall 222 of an observation housing 224 similar in many respects to housing 22 of opthalmoscope 10 (Figs. 1 and 2). Housing 224 has a front opening 226 to allow light to enter the housing and strike three reflecting surfaces 228, 230 and 232 (Fig. 14). These reflecting surfaces are formed by mirrors or the faces of a prism as described above with respect to Figs. 17 and 18. In the alternative, surfaces 228, 230 and 232 can be formed in the same manner as surfaces 28 and 56 of opthalmoscope 10.

Light reflected from surfaces 228 and 230 is directed laterally toward opposed sides of housing 224 and strikes reflecting surfaces (not shown) which reflect the light rearwardly through oculars 234, only one of which is shown in Fig. 13. Light then passes into the eyes of the wearer of opthalmoscope 210 to permit binocular viewing forwardly of housing 224.

A camera 236 is mounted on the upper end of a tube 238 secured to and extending upwardly from housing 220. Preferably, tube 238 contains the Keplerian lens assembly shown in Fig. 16 so that camera 236 will not need a lens assembly in its normal mount. In the alternative, tube 238 can have the Galilean lens assembly 21 and camera 236 can have the lens assembly 25 as shown in Fig. 16a.

Tube 238 is aligned with a hole in the top of housing 222 so that light reflected upwardly by reflecting surface 232, will pass through tube 238, through the lens assembly carried thereby, and onto the film plane of the camera when the shutter of the camera is tripped. The camera will be constructed in the manner described above with respect to Fig. 7.

A light source 240 in the form of an electrically actuated halogen bulb (Fig. 15) is mounted in a tube 242 coupled to and extending laterally from one side 234 of a second housing 236 mounted on the front wall of housing 222. Housing 242 also contains a spherical mirror 244 for reflecting light from light bulb 240 forwardly and toward and through collimating lens assembly 246. Mirror 244 is a dichroic coated mirror so that the infra-red radiation from light bulb 240 will pass through mirror 244 and exit from tube 242 in the direction of arrow 248. Thus, only the cold light generated by light bulb 240 will be reflected by mirror 244. An infra-red dichroic filter 250 is mounted on tube 242 above light bulb 240 to allow the infra-red radiation rising from light bulb 240 to pass out of tube 242 along the direction of arrow 252. An infra-red absorption filter 254 is forwardly of lens assembly 246.

Electrical leads 256 are electrically coupled to the filament of light bulb 240 to energize the same when a power supply is coupled to the leads. When the light bulb is energized, light therefrom will be directed forwardly through lens assembly 246 and the light will enter housing 236 and strike a first inclined mirror 258. The light reflected from mirror 258 will strike a second inclined mirror 260 and the light reflected from mirror 260 will be reflected forwardly to the eye of the patient and enter the eye for illuminating the fundus of the eye.

The light reflected from the fundus of the eye will return toward opthalmoscope 210 and enter opening 226 of housing 224. Some of the returning light will strike reflecting surfaces 228 and 230 and be reflected laterally therefrom for binocular viewing through oculars 234 of housing 224. The remaining light will strike reflecting surface 232 and be reflected upwardly through housing 222, tube 238 and into camera 236. The light will pass to the film plane of the camera when the shutter of the camera is tripped.

Opthalmoscope 210 provides an extremely lightweight construction by virtue of the use of the light source near the front of the opthalmoscope. The light source itself is small in size and lightweight in construction, it has means for eliminating infra-red energy before such energy has a chance to reach the fundus of the eye, and eliminates the need for a fiber optics bundle at the side of head assembly 212.

Many of the structural parts of the various embodiments of the opthalmoscopes of the present invention can be made from molded parts. The opthalmoscope 210 is a preferred construction because it is compact is size, it is lightweight in construction, and has a minimum number of parts to reduce production costs as well as overall weight. It can easily be maintained and put on and taken off with a minimum of effort and without undue trouble.

IN THE CLAIMS:

1. A binocular indirect ophthalmoscope comprising: a support adapted to be worn on the head of the user of the ophthalmoscope, said support having a front portion; a light source carried by the support and having means for directing light forwardly thereof so that the light can enter an eye of a patient; means on the support near the front portion thereof for receiving light reflected from the eye of a patient and for directing at least a first part of the reflected light into the eyes of the user; a camera coupled to the support near said receiving and directing means; and means coupled with the camera for directing at least a second part of the reflected light toward and into the camera to permit film in the camera to be exposed with the image of the eye of the patient.

2. An ophthalmoscope as set forth in Claim 1, wherein the camera is above the receiving and directing means.

3. An ophthalmoscope as set forth in Claim 1, wherein the camera is at one side of the receiving and directing means.

4. An ophthalmoscope as set forth in Claim 1, wherein the light source is mounted on the support near the rear end thereof, there being a fiber optics bundle for directing the light forwardly of the light source.

5. An ophthalmoscope as set forth in Claim 1, wherein the light source is above the receiving and directing means.

6. An ophthalmoscope as set forth in Claim 1, wherein the receiving and directing means includes a housing having a front opening, a beam splitter in the housing rearwardly of the opening, and means defining a pair of reflecting surfaces in the housing near the sides thereof for directing the beams split by the beam splitter into the eyes of the user, said means for directing at least said second part of the reflected light including means permitting the incoming rays to pass toward and into the camera.

7. An ophthalmoscope as set forth in Claim 6, wherein the camera is coupled to the housing near one side thereof, the reflecting surface near said one side of the housing including a half-silvered mirror to permit light to simultaneously pass into the camera and to be reflected by said one mirror.

8. An ophthalmoscope as set forth in Claim 1, wherein the camera has an electrically actuated speed mechanism and an electrically actuated aperture mechanism, and means coupled with the camera for selectively applying a voltage to said mechanism to adjust the same.

9. An ophthalmoscope as set forth in Claim 1, wherein the directing means for said second part of the reflected light includes a tube coupled to the camera, and a lens assembly in the tube.

10. An ophthalmoscope as set forth in Claim 1, wherein said light source includes a housing having means for mounting the same on said receiving and directing means, said light source further including a light bulb in the housing, and means coupled with the housing for dissipating at least a portion of the infra-red radiation from the light bulb before the light from the light bulb is directed to the eye of a patient.

FIG. 1

**FIG. 2**

**FIG. 3**

FIG. 4

0048181

FIG. 5

FIG. 6

FIG. 7

POWER SUPPLY

FIG. 8

**FIG. 9**

10

13

17

14

14

82

80

84

18a

86

92

88

91

56a

22a

**FIG. 10**

84

86

91

87

91

88

37

86

FIG. 11

112

110

116

114

120

145

118

126   127   122

124   124

116

FIG. 12

144

132   134   21

130

127   136

128   138

140   142

0048181

FIG. 13

FIG. 14

FIG. 15

FIG. 16

16

27

18

29

56

37

FIG. 16a

25

16

21

18

56

37

63

57

FIG. 18

FIG. 17

63

59

59

61